# EUROPEAN PATENT APPLICATION

(11) **EP 4 806 380 A1**
(43) Date of publication of application: **16.09.2026**
(21) Application number: 24911172.5
(22) Date of filing: 25.12.2024
(51) Int. Cl.: A61B 34/30

(54) **METHOD FOR ADJUSTING MECHANICAL ARM CONFIGURATION BASED ON SINGLE-CART SINGLE-ARM SURGICAL ROBOT SYSTEM AND SINGLE-CART SINGLE-ARM SURGICAL ROBOT SYSTEM**

(30) Priority: 26.12.2023 CN 202311819732
(71) Applicant: Ronovo (Shanghai) Medical Science and Technology Ltd., Shanghai 200233 (CN)
(72) Inventor: DONG, Tianlai, Shanghai 200233 (CN); ZHAO, Leidi, Shanghai 200233 (CN); JIN, Xin, Shanghai 200233 (CN); MAO, Ying, Shanghai 200233 (CN)
(74) Representative: Cabinet Beaumont
(86) International application number: PCT/CN2024/142127
(87) International publication number: WO 2025/140248

(57) **Abstract**

The present application relates to the technical field of medical devices and, more particularly, to a method for adjusting a mechanical arm configuration based on a single-cart single-arm surgical robot system and a single-cart single-arm surgical robot system. The method includes: step S1, triggering the movement of a mechanical arm by employing a button-combination triggering mode; step S2, reading a target pose corresponding to a button combination and calculating a planned movement trajectory of the mechanical arm; and step S3, moving the mechanical arm along the planned movement trajectory to reach the target pose. Thus, in the method for adjusting the mechanical arm configuration based on the single-cart single-arm surgical robot system provided in the present application, through the operation of the button combination, the pose of the mechanical arm can be conveniently adjusted, and safety can be ensured, thereby effectively avoiding collisions between the mechanical arm and the exterior or the mechanical arm. In this manner, when a collision occurs, the movement of the mechanical arm can be stopped immediately.

## Description

This application claims priority to Chinese Patent Application No. 202311819732.9 filed on Dec. 26, 2023, the disclosure of which is incorporated herein by reference in its entirety.

### TECHNICAL FIELD

The present application relates to the technical field of medical devices and, more particularly, to a method for adjusting a mechanical arm configuration based on a single-cart single-arm surgical robot system and a single-cart single-arm surgical robot system.

### BACKGROUND

During robotic surgery, a bedside cart of a surgical robot system is placed alongside an operating table. Typically, the cart includes a cart base and a mechanical arm, and the mechanical arm of the cart carries and drives a dedicated surgical instrument to perform a surgical operation. Before the surgical operation and after completion of the surgery, multiple adjustments are performed on the joints of the mechanical arm of the bedside cart to complete preoperative preparation and postoperative withdrawal operations in an operating room. Various mechanical arm poses are employed in these processes, such as storage, docking, sterile barrier installation, and specific poses tailored for different surgical procedures.

In a conventional single-cart multi-arm surgical robot system, due to a large footprint and a relatively simple configuration of the mechanical arm, a specific storage pose and a recommended pose tailored for specific surgery are preset by the manufacturer before delivery. When adjusting to the storage pose, a user needs to select a corresponding function on an operation screen located behind the cart so as to fold or unfold the mechanical arm to the specific pose. When adjusting to a pose for installing a sterile barrier or a pose tailored for specific surgery, the user needs to adjust the mechanical arm by dragging to complete the adjustment of the mechanical arm.

Compared with the conventional single-cart multi-arm surgical robot system described above, a single-cart single-arm surgical robot system has a more flexible mechanical arm joint configuration and mechanical arm configuration and can present more abundant placement poses. According to surgical requirements, placement poses of the system beside the operating table are also more diverse so that multiple carts or a single cart can be flexibly employed in practical applications.

Therefore, the adjustment requirements of the mechanical arm of the single-cart single-arm surgical robot system are more numerous and more complex than those of the single-cart multi-arm surgical robot system. The one-click folding function or the dragging placement function that is employed in the single-cart multi-arm surgical robot system cannot effectively satisfy the requirement of simplifying the surgical process.

### SUMMARY

An object of the present application is to provide a method for adjusting a mechanical arm configuration based on a single-cart single-arm surgical robot system and a single-cart single-arm surgical robot system, thereby solving the problem in the existing art that adjustment of the mechanical arm of the single-cart multi-arm surgical robot system is relatively complex.

To achieve the above object, the present application provides a method for adjusting a mechanical arm configuration based on a single-cart single-arm surgical robot system. The method includes the steps below.

In step S1, a movement of a mechanical arm is triggered by employing a button-combination triggering mode.

In step S2, a target pose corresponding to a button combination is read, and a planned movement trajectory of the mechanical arm is calculated.

In step S3, the mechanical arm is moved along the planned movement trajectory to reach the target pose.

In some embodiments, a button type of the button combination in step S1 includes a physical button, a tactile button, and a virtual button.

In some embodiments, the button-combination triggering mode in step S1 includes pressing at least two buttons simultaneously or pressing at least two buttons sequentially.

In some embodiments, a button form of the button combination in step S1 includes buttons on the mechanical arm, buttons on a switch panel of a cart, and buttons on a touchscreen of the cart.

In some embodiments, the button-combination triggering mode further includes the following.

After the first button in the button combination is pressed, a wait of a preset time is performed, and if all other buttons in the button combination are pressed within the preset time, a button-combination function is triggered after the preset time has elapsed.

In some embodiments, the target pose includes a storage pose, a sterile barrier installation pose, an inverted placement pose, and a forward placement pose.

When the target pose is the storage pose, a triggering logic of the button combination is pressing one of an instrument clutch button, a port clutch coarse adjustment button, or a port clutch fine adjustment button and pressing a null-space downward adjustment button.

When the target pose is the sterile barrier installation pose, the triggering logic of the button combination is pressing the instrument clutch button and a null-space upward adjustment button.

When the target pose is the inverted placement pose, the triggering logic of the button combination is pressing the port clutch coarse adjustment button and the null-space upward adjustment button.

When the target pose is the forward placement pose, the triggering logic of the button combination is pressing the port clutch fine adjustment button and the null-space upward adjustment button.

In some embodiments, in a storage and transport stage, the mechanical arm is controlled to reach the storage pose by employing the button-combination triggering mode.

In a surgical preparation stage, the mechanical arm is controlled to reach the sterile barrier installation pose by employing the button-combination triggering mode for installing a sterile barrier.

In a beginning stage of a surgery, based on a type and a manner of the surgery, the mechanical arm is controlled to reach a specific placement pose by employing the button-combination triggering mode, and the specific placement pose includes the inverted placement pose or the forward placement pose.

In an end stage of the surgery, the mechanical arm is controlled to return to the storage pose by employing the button-combination triggering mode.

In some embodiments, a real-time path planning algorithm is employed in step S2, and the real-time path planning algorithm calculates a potential collision path trajectory to obtain the planned movement trajectory of the mechanical arm.

In some embodiments, step S3 further includes, during the movement of the mechanical arm along the planned movement trajectory, continuously determining whether a button is released, and stopping the movement of the mechanical arm in response to the button being released.

In some embodiments, the target pose includes the storage pose.

When the mechanical arm is in the storage pose, a projection of the mechanical arm on the ground is entirely located within a range of a cart base.

In some embodiments, the target pose includes the sterile barrier installation pose.

When the mechanical arm is in the sterile barrier installation pose, all joint links of the mechanical arm are in the following state:

All joint links of the mechanical arm that are protected by a sterile barrier are within a target height range from the ground; and/or an angle between every two adjacent joint links of the mechanical arm is larger than a right angle.

In some embodiments, the target pose includes the inverted placement pose.

When the mechanical arm is in the inverted placement pose, an included angle between a parallelogram joint axis and the ground is negative, and a pointing direction of an instrument is opposite to a pointing direction from the cart base to a patient.

In some embodiments, the included angle between the parallelogram joint axis and the ground ranges from -5° to -30°.

In some embodiments, the target pose includes the forward placement pose.

When the mechanical arm is in the forward placement pose, the included angle between the parallelogram joint axis and the ground is positive, and the pointing direction of the instrument is the same as the pointing direction from the cart base to the patient.

In some embodiments, the included angle between the parallelogram joint axis and the ground ranges from 0° to 30°.

In some embodiments, in step S3, during the movement of the mechanical arm, an operating state is prompted in a manner of light or sound.

In order to achieve the above object, the present application provides a single-cart single-arm surgical robot system. The system employs the above method for adjusting the mechanical arm configuration based on the single-cart single-arm surgical robot system and includes at least a mechanical arm, a control device, and a triggering device.

The triggering device is configured to trigger the movement of the mechanical arm by employing the button-combination triggering mode.

The control device is configured to read the target pose corresponding to the button combination and calculate the planned movement trajectory of the mechanical arm.

The mechanical arm is configured to be moved along the planned movement trajectory to reach the target pose.

In some embodiments, the target pose includes a storage pose, a sterile barrier installation pose, and a specific placement pose.

In a storage and transport stage, the triggering device employs the button-combination triggering mode to enable the control device to control the mechanical arm to reach the storage pose.

In a surgical preparation stage, the triggering device employs the button-combination triggering mode to enable the control device to control the mechanical arm to reach the sterile barrier installation pose for installing a sterile barrier.

In a beginning stage of a surgery, based on a type and a manner of the surgery, the triggering device employs the button-combination triggering mode to enable the control device to control the mechanical arm to reach the specific placement pose, and the specific placement pose includes an inverted placement pose or a forward placement pose.

In an end stage of the surgery, the triggering device employs the button-combination triggering mode to enable the control device to control the mechanical arm to return to the storage pose.

In the method for adjusting the mechanical arm configuration based on the single-cart single-arm surgical robot system and the single-cart single-arm surgical robot system that are provided in the present application, the pose of the mechanical arm can be conveniently adjusted through the operation of the button combination, thereby reducing the risk of extrusion and collision of the mechanical arm and improving safety.

### BRIEF DESCRIPTION OF DRAWINGS

The above and other features, characteristics, and advantages of the present application will become more apparent from the following description of embodiments in conjunction with drawings, in which the same reference numerals throughout the drawings represent the same features.
FIG. 1 is a step diagram of a method for adjusting a mechanical arm configuration based on a single-cart single-arm surgical robot system according to an embodiment of the present application.
FIG. 2 is a positional diagram of buttons of a mechanical arm according to an embodiment of the present application.
FIG. 3 is a flowchart of movement path planning of a mechanical arm according to an embodiment of the present application.
FIG. 4A is a pose diagram of a cart in a storage pose according to an embodiment of the present application.
FIG. 4B is a pose diagram of a cart in a sterile barrier pose according to an embodiment of the present application.
FIG. 5A is a pose diagram of a cart in an inverted placement pose according to an embodiment of the present application.
FIG. 5B is a pose diagram of a cart in a forward placement pose according to an embodiment of the present application.
FIG. 6 is a diagram of a cart reaching an abdominal entry hole to perform an operation in an inverted placement pose of a mechanical arm according to an embodiment of the present application.
FIG. 7 is a top view of a cart reaching an abdominal entry hole to perform an operation in an inverted placement pose of a mechanical arm according to an embodiment of the present application.
FIG. 8A is a diagram of a first placement pose of a mechanical arm according to an embodiment of the present application.
FIG. 8B is a diagram of a second placement pose of a mechanical arm according to an embodiment of the present application.
FIG. 8C is a diagram of a third placement pose of a mechanical arm according to an embodiment of the present application.
FIG. 9A is a diagram of a first placement pose of a mechanical arm in an inverted placement pose according to an embodiment of the present application.
FIG. 9B is a diagram of a second placement pose of a mechanical arm in an inverted placement pose according to an embodiment of the present application.
FIG. 9C is a diagram of a movement range of a mechanical arm in an inverted placement pose according to an embodiment of the present application.
FIG. 10 is a diagram illustrating the operation of a cart when a mechanical arm is in a forward placement pose according to an embodiment of the present application.
FIG. 11 is a top view of the operation of a cart when a mechanical arm is in a forward placement pose according to an embodiment of the present application.
FIG. 12A is a diagram of a first placement pose of a mechanical arm in a forward placement pose according to an embodiment of the present application.
FIG. 12B is a diagram of a second placement pose of a mechanical arm in a forward placement pose according to an embodiment of the present application.
FIG. 12C is a diagram of a movement range of a mechanical arm in a forward placement pose according to an embodiment of the present application.

### Reference list

- 10: cart button group
- 21: port clutch coarse adjustment button
- 22: port clutch fine adjustment button
- 23: instrument clutch button
- 24: null-space upward adjustment button
- 25: null-space downward adjustment button
- 26: fixed point
- 31: abdominal entry hole
- 32: surgical operating region
- 33: abdominal cavity region of a patient
- 41: first joint link
- 42: second joint link
- 43: third joint link
- 44: fourth joint link
- 45: fifth joint link
- 46: sixth joint link

### DETAILED DESCRIPTION

To illustrate the object, technical solutions, and advantages of the present application more clearly, the present application is further described below in detail in conjunction with the drawings and embodiments. It is to be understood that the embodiments described herein are intended to explain and not to limit the present application.

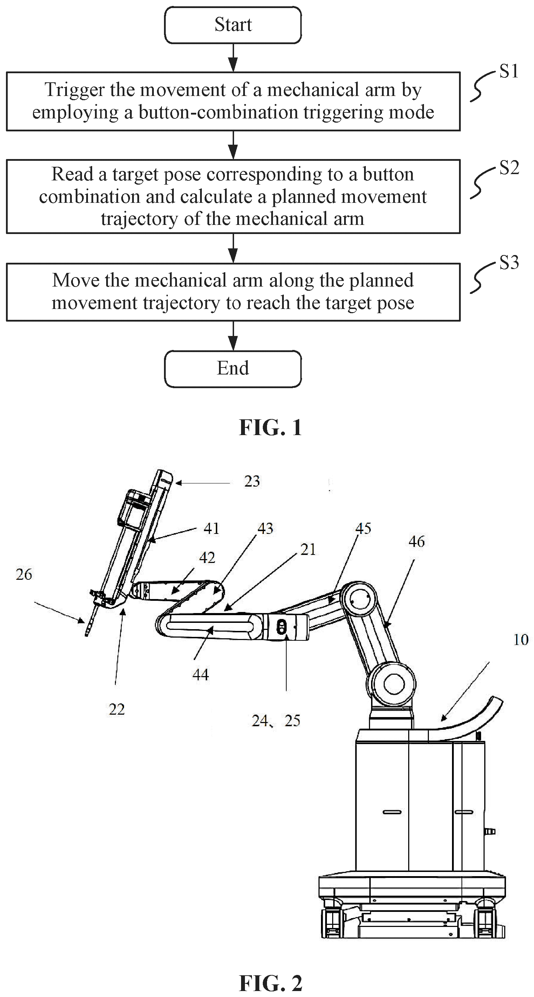

FIG. 1 is a step diagram of a method for adjusting a mechanical arm configuration based on a single-cart single-arm surgical robot system according to an embodiment of the present application. As shown in FIG. 1, the method for adjusting the mechanical arm configuration based on the single-cart single-arm surgical robot system provided in the present application includes the following steps.

In step S1, the movement of a mechanical arm is triggered by employing a button-combination triggering mode.

In step S2, a target pose corresponding to a button combination is read, and a planned movement trajectory of the mechanical arm is calculated.

In step S3, the mechanical arm is moved along the planned movement trajectory to reach the target pose.

The method for adjusting the mechanical arm configuration based on the single-cart single-arm surgical robot system provided in the present application may be applied not only to multiple single-cart single-arm surgical robot systems but also to one single-cart single-arm surgical robot system.

These steps are described in detail below. It should be understood that within the scope of the present application, the above technical features of the present application and technical features specifically described below (such as embodiments) can be combined with each other to form a preferred technical solution.

In step S1, the movement of the mechanical arm is triggered by employing the button-combination triggering mode.

FIG. 2 is a positional diagram of buttons of a mechanical arm according to an embodiment of the present application. As shown in FIG. 2, there are five buttons on the mechanical arm, including a port clutch coarse adjustment button 21, a port clutch fine adjustment button 22, an instrument clutch adjustment button 23, a null-space upward adjustment button 24, and a null-space downward adjustment button 25. It should be noted that, though the buttons in this embodiment are physical buttons with tangible properties, these buttons can alternatively be replaced by virtual buttons existing in a graphical user interface without physical forms.

Moreover, to enhance operational intuitiveness, the positions of these buttons are arranged near corresponding movement joints/arms after consideration, thereby facilitating user operation.

As shown in FIG. 2, the mechanical arm includes a first joint link 41, a second joint link 42, a third joint link 43, a fourth joint link 44, a fifth joint link 45, and a sixth joint link 46.

The first joint link 41 is connected to the second joint link 42.

The second joint link 42 is connected to both the first joint link 41 and the third joint link 43.

The third joint link 43 is connected to both the second joint link 42 and the fourth joint link 44.

The fourth joint link 44 is connected to both the third joint link 43 and the fifth joint link 45.

The fifth joint link 45 is connected to both the fourth joint link 44 and the sixth joint link 46.

The sixth joint link 46 is connected to both the fifth joint link 45 and a cart base.

The positions of the five buttons shown in FIG. 2 are as follows.

The null-space upward adjustment button 24 or the null-space downward adjustment button 25 is arranged at the end of the fourth joint link 44.

The instrument clutch button 23 is arranged above the first joint link 41.

The port clutch fine adjustment button 22 is arranged below the first joint link 41.

The port clutch coarse adjustment button 21 is arranged at the middle position of the fourth joint link 44.

In the existing art, to adjust the mechanical arm to a desired pose, the above adjustment buttons are required to be repeatedly adjusted. This not only consumes a large amount of time, but also results in low adjustment accuracy. In addition, a user needs a relatively long learning curve and strong spatial imagination. During the autonomous movement of the mechanical arm, a risk of collision with surrounding personnel or a device, or of extrusion or pinching of the human body (a finger or skin) or a surgical accessory in an operating room (such as a sterile barrier of the mechanical arm) exists.

In the method for adjusting the mechanical arm configuration based on the single-cart single-arm surgical robot system provided in the present application, the button-combination triggering mode is employed, in which at least two buttons are pressed simultaneously or sequentially. The buttons require the hands of a user to move along with the movement of the mechanical arm, that is, both hands remain on the buttons without leaving. Consequently, unlike a conventional dragging approach, the movement direction of the mechanical arm is not determined by the direction in which the user drags. In the conventional dragging approach, adjusting the mechanical arm typically refers to directly dragging button parts or parameters of the mechanical arm through a graphical user interface (GUI), a button, or other interactive means to change the pose, the position, or other characteristics of the mechanical arm in real time.

In this embodiment, the movement of the mechanical arm is controlled by one button combination. For example, as shown in FIG. 2, one button of the button combination is located on the fourth joint link 44 at the proximal end of the mechanical arm, which is either the null-space upward adjustment button 24 or the null-space downward adjustment button 25; and the other button of the button combination is located on the first joint link 41 at the distal end of the mechanical arm, which is either the instrument clutch button 23 or the port clutch fine adjustment button 22, or the other button is the port clutch coarse adjustment button 21 at the middle of the fourth joint link 44.

When the user uses the button combination, both hands grasp the mechanical arm, allowing the user to perceive the movement of the mechanical arm through tactile feedback and to visually observe potential collisions of the mechanical arm. Since the user operates the mechanical arm with both hands and stands beside the mechanical arm, pressing buttons requires both hands to contact the mechanical arm, which can reduce the risk of fingers being inadvertently pinched by the mechanical arm, thereby improving safety.

The process of adjusting the mechanical arm to reach a bedside placement pose involves a large-scale movement of the mechanical arm. When the user presses the button combination, the mechanical arm can autonomously complete the pose adjustment. Specifically, the user presses two buttons simultaneously. To ensure an operation within the field of view of the user and to avoid a collision with obstacles in the external environment, the buttons keep being pressed until a target position is reached. During the mechanical movement, the user needs to monitor the mechanical arm to prevent extrusion or collision of the mechanical arm with personnel, devices, or accessories. Furthermore, sufficient safety is also provided to ensure that the mechanical arm can be immediately stopped if any unexpected condition occurs.

In this embodiment, the control logic for triggering the movement of the mechanical arm employs the button-combination triggering mode. However, any button in the button combination, if pressed individually, can control the movement of a corresponding joint of the mechanical arm. To avoid possible confusion from multiple simultaneous movements, the present application provides the following triggering logic of the button combination.

After the first button of the button combination is pressed, a wait of a preset time is performed, and if all other buttons in the button combination are pressed within the preset time, a button-combination function is triggered after the preset time has elapsed. Within the preset time, the mechanical arm does not initiate the autonomous movement.

To be more specific, after any button is pressed, the mechanical arm does not immediately start moving but waits for a short interval (such as 0.2 seconds). If any button combination is pressed during this waiting process, the system fulfills the button-combination function after the waiting period ends.

Furthermore, button types of the button combination include, but are not limited to, a physical button, a tactile button, and a virtual button capable of detecting a physical press.

In this embodiment, all the buttons are triggered by pressing physical buttons to ensure that a finger/palm of a surgeon grasps the robot while the surgeon triggers the buttons, thereby improving safety during the movement of the robot. The autonomous movement of the mechanical arm is immediately stopped when any button is released.

Furthermore, the button combination may be multiple buttons that are located at any positions on the mechanical arm and facilitate an operation by an assistant.

In this embodiment, the autonomous movement of the mechanical arm may also be controlled by employing a button combination that is not on the mechanical arm. This control approach includes, but is not limited to, buttons on a switch panel of the cart base or a button function on a touchscreen of a cart, such as a cart button group 10 shown in FIG. 2.

In step S2, the target pose corresponding to the button combination is read, and the planned movement trajectory of the mechanical arm is calculated.

During the process of waiting for the button combination to be triggered, an industrial computer in a patient surgical platform starts to calculate the movement trajectory.

During the large-scale movement of the mechanical arm, self-collision between joint links of the mechanical arm may occur. Therefore, during the process in which the button combination controls the mechanical arm to autonomously move, a real-time path planning function is employed.

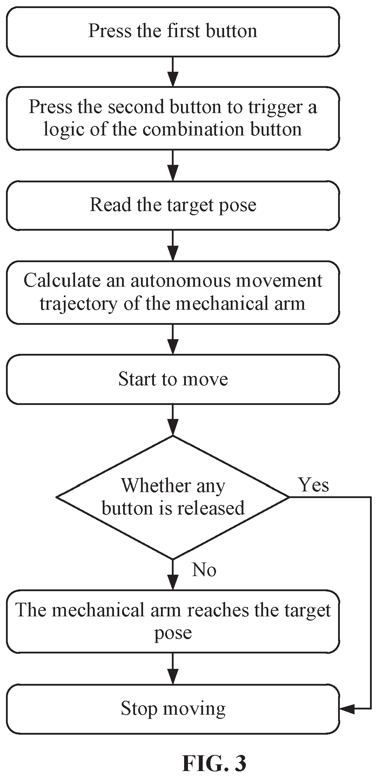

FIG. 3 is a flowchart of movement path planning of a mechanical arm according to an embodiment of the present application. As shown in FIG. 3, each time the button combination of the mechanical arm is pressed, that is, after the first button and a second button are sequentially pressed, the computer reads the target pose according to a logic of the button combination, executes a mechanical-arm path planning algorithm, and detects a potential collision path so that the trajectory for avoiding self-collisions can be calculated and updated to complete the movement of the mechanical arm autonomously. The mechanical arm is moved along the planned movement trajectory until it reaches the target pose. During the movement, whether any button in the button combination is released is continuously determined. If any button is released, the calculation of the planned movement trajectory of the mechanical arm and/or the movement of the mechanical arm is stopped.

The mechanical-arm path planning algorithm comprehensively considers factors of the mechanical arm, such as the movement range, speed, and acceleration of the mechanical arm, to plan an optimal movement trajectory from the starting point to the end point.

In this embodiment, the target pose of the mechanical arm includes, but is not limited to, a storage pose, a sterile barrier installation pose, and a placement pose for each different surgical procedure (an inverted placement pose and a forward placement pose).

The above poses of the mechanical arm may be poses configured by default before delivery of the robot, poses designed by a user according to an operating habit, and poses recommended by the system according to a surgical procedure selected by the user.

The target pose is stored in the industrial computer of a cart or in a robot system.

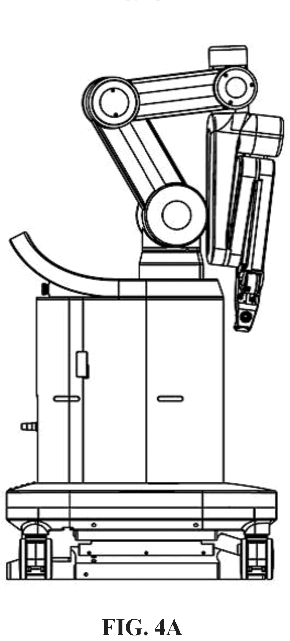

In this embodiment, the poses of the mechanical arm include, but are not limited to, the storage pose, the sterile barrier installation pose, the inverted placement pose, and the forward placement pose, as shown in FIGS. 4A to 5B.

In the storage and transport stage, the mechanical arm is controlled to reach the storage pose by employing the button-combination triggering mode.

In the surgical preparation stage, the mechanical arm is controlled to reach the sterile barrier installation pose by employing the button-combination triggering mode for installing a sterile barrier.

In the beginning stage of a surgery, based on a type and a manner of the surgery, the mechanical arm is controlled to reach a specific placement pose by employing the button-combination triggering mode, and the specific placement pose includes the inverted placement pose and the forward placement pose.

In the end stage of the surgery, the mechanical arm is controlled to return to the storage pose by employing the button-combination triggering mode.

FIG. 4A is a pose diagram of a cart in a storage pose according to an embodiment of the present application. As shown in FIG. 4A, when the mechanical arm is switched to the storage pose, the triggering logic of the button combination is pressing one of the instrument clutch button 23, the port clutch coarse adjustment button 21, or the port clutch fine adjustment button 22 and pressing the null-space downward adjustment button 24.

When the mechanical arm is in the storage pose, the projection of the mechanical arm on the ground is entirely located within the range of the cart base. In this case, the occupied footprint is minimized.

After the sterile barrier is removed after the surgery, no matter where the mechanical arm of the cart is located, the mechanical arm may be adjusted to the storage pose by pressing any one of the instrument clutch button, the port clutch coarse adjustment button, or the port clutch fine adjustment button and pressing the null-space downward adjustment button, thereby preventing the mechanical arm from pinching fingers or avoiding an accident caused by misoperation.

In the existing art, the operation of switching to the storage pose by the dragging approach or pressing null-space adjustment buttons requires frequent adjustments, and the movement is not intuitive. However, in this embodiment, switching between the storage pose and other poses can be conveniently accomplished. This switching process is more intuitive and can be completed quickly and accurately.

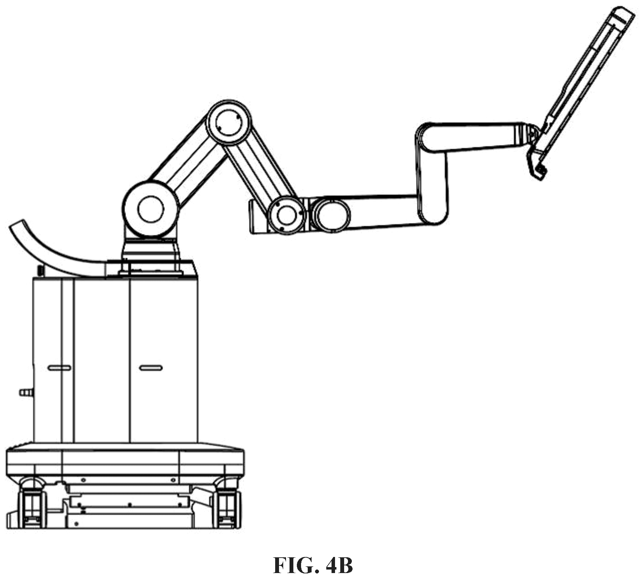

FIG. 4B is a pose diagram of a cart in a sterile barrier pose according to an embodiment of the present application. As shown in FIG. 4B, when the mechanical arm is switched to the sterile barrier installation pose, the triggering logic of the button combination is: pressing the instrument clutch button 23 and the null-space upward adjustment button 24.

When the cart is placed in an open region of the operating room, the user may press the button combination to extend the mechanical arm to the sterile barrier installation pose, facilitating the installation of a sterile barrier.

In the sterile barrier installation pose, all joint links of the mechanical arm are in the following state.

All joint links of the mechanical arm that are protected by the sterile barrier are within a target height range from the ground; and/or an angle between every two adjacent joint links of the mechanical arm is larger than a right angle.

In this embodiment, all joint links of the mechanical arm that are protected by the sterile barrier are within a height range of 0.9 m to 1.5 m from the ground.

The design of the sterile barrier installation pose can prevent an acute angle between the joint links or a pose that easily causes the sterile barrier to be stuck. This pose can facilitate the installation or removal of the sterile barrier by the user.

The placement poses of the mechanical arm for different surgical procedures include a pose in which the mechanical arm is placed facing forward and a pose in which the mechanical arm is placed in an inverted orientation. These two placement poses are selected and determined according to a specific surgical procedure.

Since a surgical robot system with a single-arm configuration on a single cart is applicable to abdominal surgery, thoracic surgery, and pelvic surgery, and operating regions and surgical operations therefor differ, it is necessary to reasonably select the placement pose of the cart during surgery according to the actual placement of a patient, an operating region, and surgical operating habits of the user.

During the surgery, to better match the position of the patient lying on an operating table with the surgical operating region, the placement poses of carts and the combination of multiple carts need to be carefully selected. Moreover, to achieve the optimal placement poses of the mechanical arms, appropriate poses may be selected and combined to reduce the risk of collisions between mechanical arms of different carts and also leave more operating space for the user, thereby ensuring a more rational layout in the operating room.

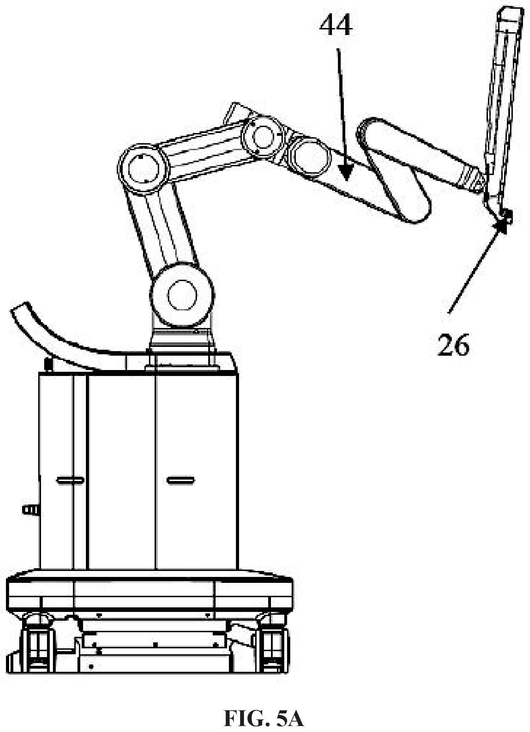

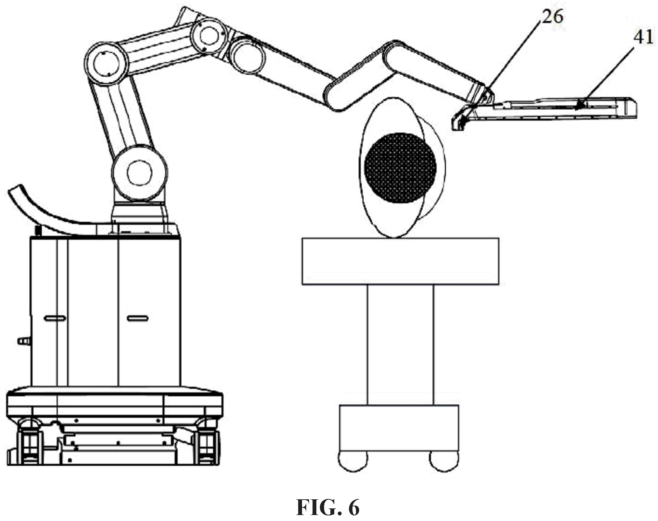

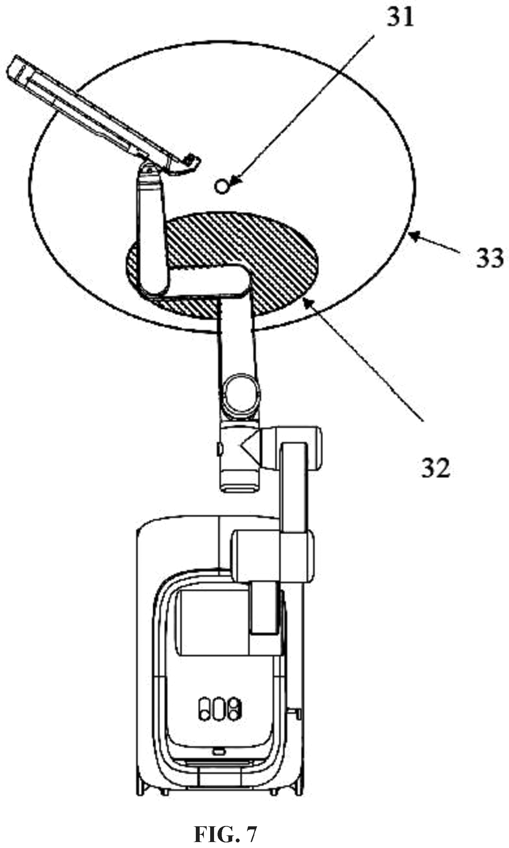

FIG. 5A is a pose diagram of a cart in an inverted placement pose according to an embodiment of the present application. FIG. 6 is a diagram of a cart reaching an abdominal entry hole to perform an operation when a mechanical arm is in an inverted placement pose according to an embodiment of the present application. FIG. 7 is a top view of a cart reaching an abdominal entry hole to perform an operation when a mechanical arm is in an inverted placement pose according to an embodiment of the present application. As shown in FIGS. 5A, 6, and 7, when the mechanical arm is in the inverted placement pose, the cart needs to cross over the patient lying on the operating table to reach the abdominal entry hole for operation. In this placement pose, the surgical operating region 32 and the region where the cart is placed are located on the same side of the abdominal entry hole 31 of the abdominal cavity region 33 of the patient. Employing this placement pose allows the cart to cross over the patient from the back side, thereby avoiding cumbersome adjustment operations.

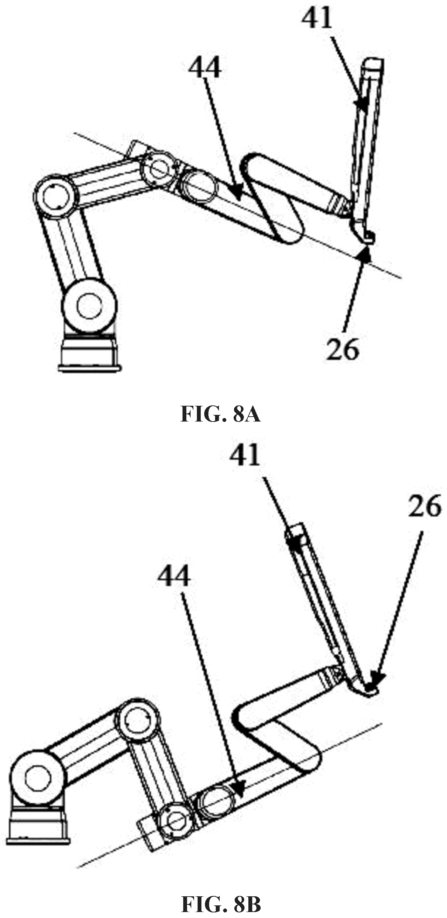

FIG. 8A is a diagram of a first placement pose of a mechanical arm according to an embodiment of the present application. As shown by the placement features in FIG. 8A, when the mechanical arm is in the inverted placement pose, an included angle between the parallelogram joint axis and the ground is negative, and the pointing direction of a surgical instrument is opposite to the pointing direction from the cart base to the patient.

In this embodiment, the first joint link 41, the second joint link 42, the third joint link 43, and the fourth joint link 44 form a parallelogram. That is, during the movement of the links, by design limits, the first joint link 41 and the third joint link 43 remain parallel, while the fourth joint link 44 and the second joint link 42 remain parallel. The parallelogram joint axis refers to the axis of the fourth joint link 44, and the first joint link 41 of the mechanical arm is used to carry the surgical instrument. Therefore, the pointing direction of the surgical instrument refers to the direction in which the surgical instrument passes through a fixed point 26 and is pointed to a target surgical region.

An included angle in the clockwise direction is defined as positive, while an included angle in the counterclockwise direction is defined as negative.

Preferably, the included angle between the parallelogram joint axis and the ground ranges from -5° to -30°. In this placement pose, relatively ample space can be provided at the position beneath the mechanical arm, thereby avoiding collisions with the body of the patient during the movement.

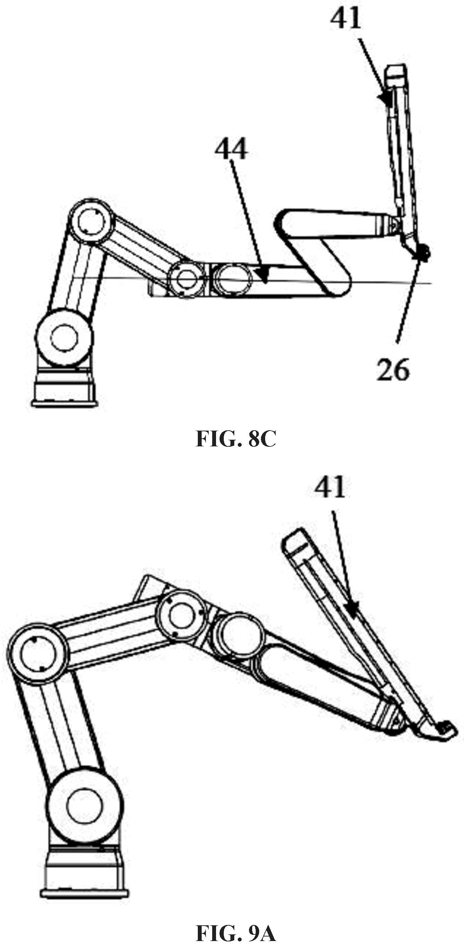

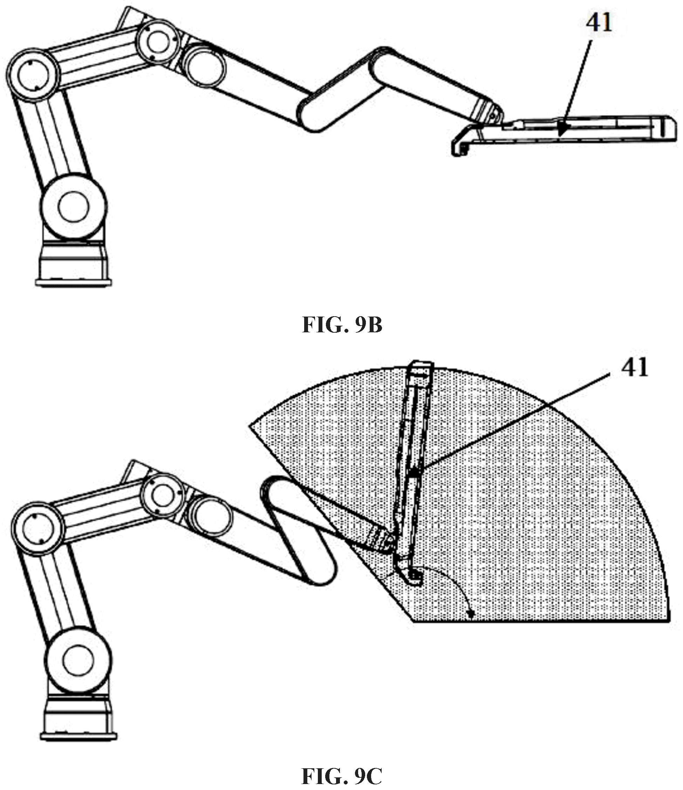

FIG. 9A is a diagram of a first placement pose of a mechanical arm in an inverted placement pose according to an embodiment of the present application. FIG. 9B is a diagram of a second placement pose of a mechanical arm in an inverted placement pose according to an embodiment of the present application. FIG. 9C is a diagram of a movement range of a mechanical arm in an inverted placement pose according to an embodiment of the present application. As shown in FIGS. 9A to 9C, an angle between the first joint link 41 of the mechanical arm and the ground ranges from 60° to 180°.

When the mechanical arm is switched to the inverted placement pose, the triggering logic of the button combination is pressing the port clutch coarse adjustment button 21 and the null-space upward adjustment button 24.

After completing the step of installing the sterile barrier, the user presses the button combination to adjust the mechanical arm to the inverted placement pose. In this pose, the user can complete cart docking at the abdominal entry hole in the inverted placement pose.

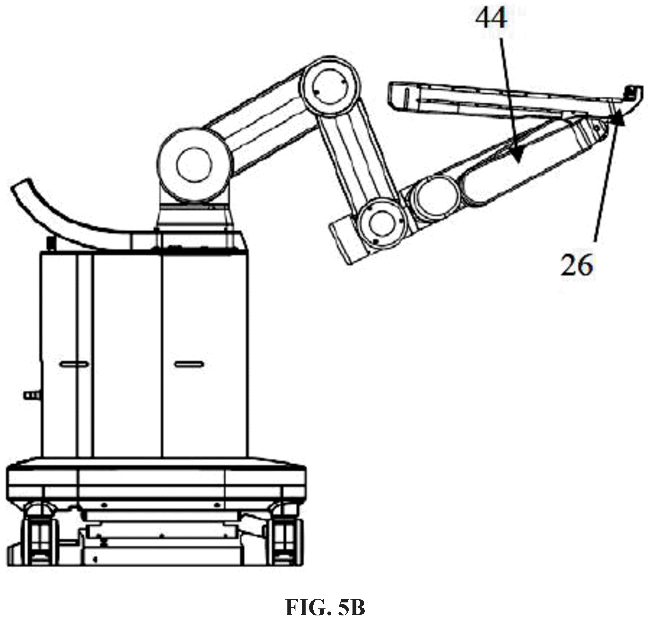

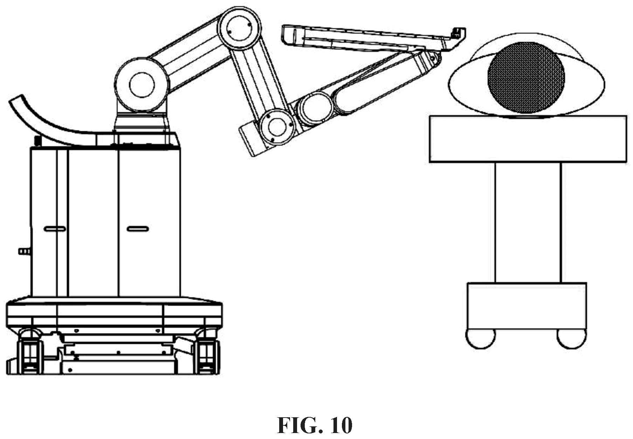

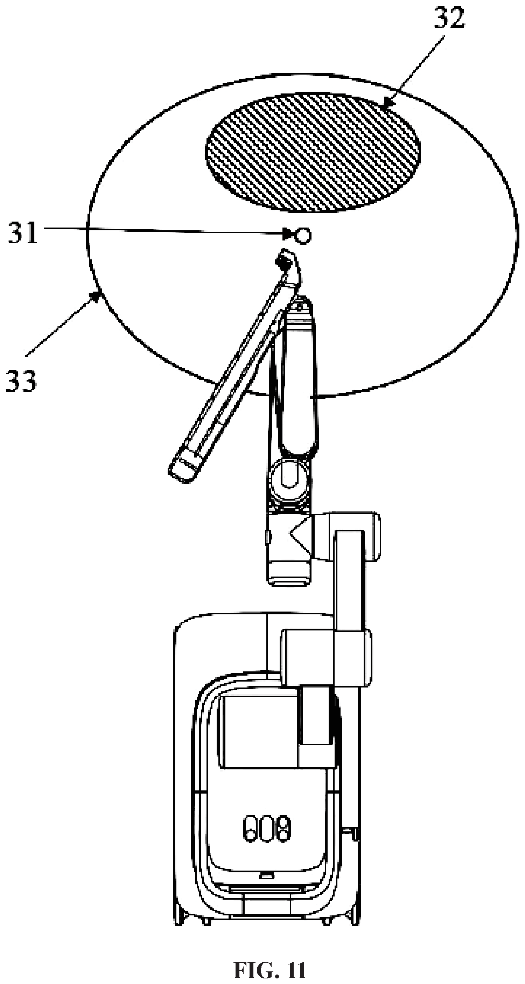

FIG. 5B is a pose diagram of a cart in a forward placement pose according to an embodiment of the present application. FIG. 10 is a diagram illustrating the operation of a cart when a mechanical arm is in a forward placement pose according to an embodiment of the present application. FIG. 11 is a top view of the operation of a cart when a mechanical arm is in a forward placement pose according to an embodiment of the present application. As shown in FIGS. 5B, 10, and 11, when the mechanical arm is in the forward placement pose, the cart base is placed right facing the abdominal entry hole on the patient so that a surgical operation is performed. In this placement pose, the surgical operating region 32 and a placement region of the cart base are located on two opposite sides of the abdominal entry hole 31 within the abdominal cavity region 33 of the patient. Employing this placement pose can prevent the cart base from being too close to the mechanical arm, which may cause self-collisions of the mechanical arm.

FIG. 8B is a diagram of a second placement pose of a mechanical arm according to an embodiment of the present application. FIG. 8C is a diagram of a third placement pose of a mechanical arm according to an embodiment of the present application. The placement features shown in FIGS. 8B and 8C each represent one of the placement poses when the mechanical arm is in the forward placement pose. When the mechanical arm is in the forward placement pose, the included angle between the parallelogram joint axis of the mechanical arm and the ground is positive, and the pointing direction of the surgical instrument is the same as the pointing direction from the cart base to the patient.

In this embodiment, the first joint link 41, the second joint link 42, the third joint link 43, and the fourth joint link 44 form a parallelogram. That is, during the movement of the links, by design limits, the first joint link 41 and the third joint link 43 remain parallel, while the fourth joint link 44 and the second joint link 42 remain parallel. The parallelogram joint axis refers to the axis of the fourth joint link 44, and the first joint link 41 of the mechanical arm is used to carry the surgical instrument. The pointing direction of the surgical instrument refers to the direction in which the surgical instrument passes through a fixed point 26 and is pointed to a target surgical region.

As shown in FIG. 8B, the included angle between the parallelogram joint axis of the mechanical arm and the ground is positive; and as shown in FIG. 8C, the included angle between the parallelogram joint axis of the mechanical arm and the ground is 0.

An included angle in the clockwise direction is defined as positive, while an included angle in the counterclockwise direction is defined as negative.

Preferably, the included angle between the parallelogram joint axis and the ground ranges from 0° to 30°. In this placement pose, relatively ample space can be provided at the position above the mechanical arm.

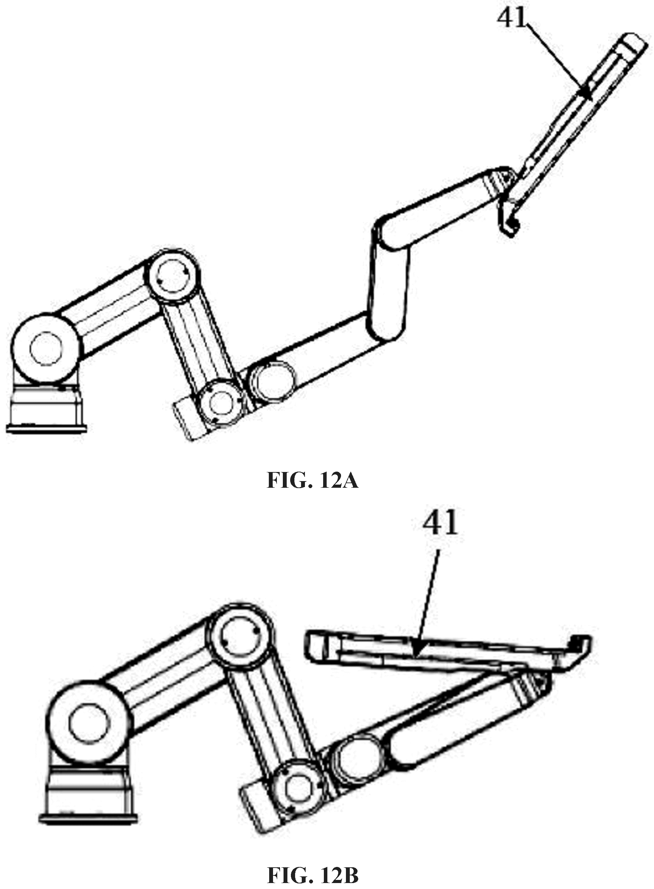

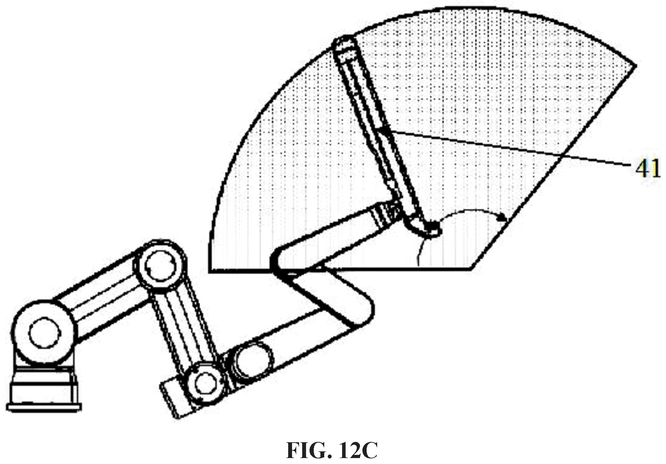

FIG. 12A is a diagram of a first placement pose of a mechanical arm in a forward placement pose according to an embodiment of the present application. FIG. 12B is a diagram of a second placement pose of a mechanical arm in a forward placement pose according to an embodiment of the present application. FIG. 12C is a diagram of a movement range of a mechanical arm in a forward placement pose according to an embodiment of the present application. As shown in FIGS. 12A to 12C, an angle between the first joint link 41 of the mechanical arm and the ground ranges from 0° to 120°.

When the mechanical arm is switched to the forward placement pose, the triggering logic of the button combination is pressing the port clutch fine adjustment button 22 and the null-space upward adjustment button 24.

After completing the step of installing the sterile barrier, the user presses the button combination to adjust the mechanical arm to the forward placement pose. In this pose, the user can complete cart docking when the cart is directly facing the abdominal entry hole.

In step S3, the mechanical arm is moved along the planned movement trajectory to reach the target pose.

The mechanical arm is provided with a real-time collision detection and path planning function during the movement. The mechanical arm detects its own movement state in real time through sensors of the mechanical arm itself and compares the movement state with the calculated planned movement trajectory. Whether a collision occurs is determined according to a preset collision detection algorithm. Once the collision is detected, the movement of the mechanical arm is stopped immediately to prevent further damage. This real-time collision detection and path planning function allows the mechanical arm to safely and efficiently perform a task in a complex environment.

After the waiting period ends, the mechanical arm starts to move, and a prompt that the mechanical arm is moving is simultaneously provided to the user in a manner of light or sound. This prompt approach can effectively remind the user to maintain safety and avoid any action that may be hazardous during the movement of the mechanical arm.

In the method for adjusting the mechanical arm configuration based on the single-cart single-arm surgical robot system provided in the present application, the mechanical arm can be adjusted conveniently and safely. Through the operation of the button combination, a preoperative pose can be conveniently adjusted, and the safety can be ensured, thereby effectively avoiding the collisions of the mechanical arm with the exterior or the mechanical arm itself. In this manner, when the collision occurs, the movement of the mechanical arm can be stopped immediately.

Although, for simplicity of explanation, the above method is illustrated and described as a series of actions, it should be understood that the method is not limited to the order of these actions. According to one or more embodiments, some actions may occur in a different order and/or concurrently with other actions illustrated and described herein or not illustrated and described but understood by those skilled in the art.

The present application further provides a single-cart single-arm surgical robot system that is applicable to both multiple single-cart single-arm surgical robot systems and a single-cart single-arm surgical robot system. The innovative mechanical arm pose configuration can be adapted to different surgical procedures, thereby facilitating various adjustments in the operating room by the user, including cart docking, storage, and sterile barrier installation.

The single-cart single-arm surgical robot system provided in the present application includes at least a mechanical arm, a control device, and a triggering device.

The triggering device triggers the movement of the mechanical arm by employing a button-combination triggering mode.

The control device reads a target pose corresponding to a button combination and calculates a planned movement trajectory of the mechanical arm.

The mechanical arm is moved along the planned movement trajectory to reach the target pose.

In some embodiments, the target pose includes a storage pose, a sterile barrier installation pose, and a specific placement pose.

In the storage and transport stage, the triggering device employs the button-combination triggering mode to enable the control device to control the mechanical arm to reach the storage pose.

In the surgical preparation stage, the triggering device employs the button-combination triggering mode to enable the control device to control the mechanical arm to reach the sterile barrier installation pose for installing a sterile barrier.

In the beginning stage of a surgery, based on the type and the manner of the surgery, the triggering device employs the button-combination triggering mode to enable the control device to control the mechanical arm to reach the specific placement pose, and the specific placement pose includes an inverted placement pose or a forward placement pose.

In the end stage of the surgery, the triggering device employs the button-combination triggering mode to enable the control device to control the mechanical arm to return to the storage pose.

The specific implementation details of the single-cart single-arm surgical robot system correspond to the above method for adjusting the mechanical arm configuration based on the single-cart single-arm surgical robot system and are therefore not repeated herein.

In the method for adjusting the mechanical arm configuration based on the single-cart single-arm surgical robot system and the single-cart single-arm surgical robot system that are provided in the present application, the pose of the mechanical arm can be conveniently adjusted through the operation of the button combination, thereby reducing the risk of extrusion and collision of the mechanical arm, and improving the safety.

As used in the present application and the claims, unless the context clearly indicates otherwise, the terms "a", "an", "one", "a kind of", and/or "the" are not intended to refer to the singular only, but may also include the plural. Generally, the terms "comprising" and "including" indicate the inclusion of the explicitly identified steps and elements, without constituting an exclusive list. The method or device may also include other steps or elements.

It is to be understood by those skilled in the art that information, signals, and data can be represented employing any of various technologies and techniques. For example, data, instructions, commands, information, signals, bits, symbols, and chips that are referred to throughout the above description may be represented by voltage, current, electromagnetic waves, magnetic fields or particles, optical fields or particles, or any combination thereof.

It is to be further appreciated by those skilled in the art that the various illustrative logic blocks, modules, circuits, and algorithmic steps that are described in connection with the embodiments disclosed herein can be implemented as electronic hardware, computer software, or a combination thereof. To clearly explain the interchangeability between hardware and software, the various illustrative assemblies, blocks, modules, circuits, and steps above are described in a generalized functional form. Whether such functionality is implemented as hardware or software depends on the particular application and the design limits imposed on the overall system. A person skilled in the art can implement the described functionality in different ways for each specific application, but such implementation choices should not be construed as departing from the scope of the present application.

The above embodiments are provided to enable those skilled in the art to implement or use the present application. Those skilled in the art can make various modifications or changes to the above embodiments without departing from the inventive concept of the present application. Therefore, the scope of protection of the present application is not limited to the above embodiments, but should cover the maximum scope consistent with the inventive features set forth in the claims.

## Claims

1. A method for adjusting a mechanical arm configuration based on a single-cart single-arm surgical robot system, comprising:
- in step S1, triggering a movement of a mechanical arm by employing a button-combination triggering mode;
- in step S2, reading a target pose corresponding to a button combination and calculating a planned movement trajectory of the mechanical arm; and
- in step S3, moving the mechanical arm along the planned movement trajectory to reach the target pose.

2. The method for adjusting the mechanical arm configuration based on the single-cart single-arm surgical robot system according to claim 1, wherein the button combination is composed of at least two buttons; and
preferably, at least one button in the button combination is a virtual button on a graphical user interface.

3. The method for adjusting the mechanical arm configuration based on the single-cart single-arm surgical robot system according to claim 2, wherein the movement of the mechanical arm is triggered after the following conditions are satisfied:
- a first button in the button combination is pressed;
- other buttons in the button combination are pressed either within a preset time after the first button is pressed or simultaneously with the first button; and
- the preset time has elapsed,
wherein preferably, after triggering the movement of the mechanical arm, stopping the movement of the mechanical arm in response to at least one button in the button combination being released.

4. The method for adjusting the mechanical arm configuration based on the single-cart single-arm surgical robot system according to any one of the preceding claims, wherein a button form of the button combination in step S1 comprises buttons on the mechanical arm, buttons on a switch panel of a cart, and buttons on a touchscreen of the cart.

5. The method for adjusting the mechanical arm configuration based on the single-cart single-arm surgical robot system according to any one of the preceding claims, wherein the mechanical arm of the single-cart single-arm surgical robot system comprises a cart base and a plurality of joint links arranged sequentially, the plurality of joint links are connected end-to-end, and a last joint link among the plurality of joint links is connected to the cart base; and
preferably, the mechanical arm of the single-cart single-arm surgical robot system comprises at least four joint links, a first joint link among the at least four joint links is provided with an instrument clutch button and/or a port clutch fine adjustment button, a fourth joint link among the at least four joint links is provided with a null-space upward adjustment button and/or a zero-space downward adjustment button, and a middle section of the fourth joint link is provided with a port clutch coarse adjustment button.

6. The method for adjusting the mechanical arm configuration based on the single-cart single-arm surgical robot system according to any one of the preceding claims, wherein the target pose comprises a storage pose, a sterile barrier installation pose, an inverted placement pose, and a forward placement pose, wherein
in response to the target pose being the storage pose, a triggering logic of the button combination is pressing one of the instrument clutch button, the port clutch coarse adjustment button, or the port clutch fine adjustment button, and pressing the null-space downward adjustment button;
in response to the target pose being the sterile barrier installation pose, the triggering logic of the button combination is pressing the instrument clutch button and the null-space upward adjustment button;
in response to the target pose being the inverted placement pose, the triggering logic of the button combination is pressing the port clutch coarse adjustment button and the null-space upward adjustment button; and
in response to the target pose being the forward placement pose, the triggering logic of the button combination is pressing the port clutch fine adjustment button and the null-space upward adjustment button.

7. The method for adjusting the mechanical arm configuration based on the single-cart single-arm surgical robot system according to any one of the preceding claims, wherein in a storage and transport stage, controlling the mechanical arm to reach a storage pose by employing the button-combination triggering mode;
in a surgical preparation stage, controlling the mechanical arm to reach a sterile barrier installation pose by employing the button-combination triggering mode for installing a sterile barrier;
in a beginning stage of a surgery, based on a type of the surgery and a manner of the surgery, controlling the mechanical arm to reach a specific placement pose by employing the button-combination triggering mode, wherein the specific placement pose comprises an inverted placement pose or a forward placement pose; and
in an end stage of the surgery, controlling the mechanical arm to return to the storage pose by employing the button-combination triggering mode.

8. The method for adjusting the mechanical arm configuration based on the single-cart single-arm surgical robot system according to any one of the preceding claims, wherein a real-time path planning algorithm is employed in step S2, and the real-time path planning algorithm calculates a potential collision path trajectory to obtain the planned movement trajectory of the mechanical arm.

9. The method for adjusting the mechanical arm configuration based on the single-cart single-arm surgical robot system according to claim 8, wherein the real-time path planning algorithm comprises detection of a potential collision path, and the real-time path planning algorithm is configured to calculate and update the planned movement trajectory for avoiding self-collisions to complete the movement of the mechanical arm autonomously.

10. The method for adjusting the mechanical arm configuration based on the single-cart single-arm surgical robot system according to any one of the preceding claims, wherein step S3 further comprises: during the movement of the mechanical arm along the planned movement trajectory, continuously determining whether a button in the button combination is released, and stopping the movement of the mechanical arm in response to the button being released.

11. The method for adjusting the mechanical arm configuration based on the single-cart single-arm surgical robot system according to claim 10, wherein the movement of the mechanical arm is followed by a movement of a hand of a user; during the movement of the mechanical arm along the planned movement trajectory, continuously determining whether the hand of the user has released the button; and in response to the hand of the user releasing the button, stopping calculation of the planned movement trajectory of the mechanical arm and/or stopping the movement of the mechanical arm.

12. The method for adjusting the mechanical arm configuration based on the single-cart single-arm surgical robot system according to any one of the preceding claims, wherein the target pose comprises a storage pose; and
in response to the mechanical arm being in the storage pose, a projection of the mechanical arm on ground is entirely located within a footprint of a cart base.

13. The method for adjusting the mechanical arm configuration based on the single-cart single-arm surgical robot system according to any one of the preceding claims, wherein the target pose comprises a sterile barrier installation pose; and
in response to the mechanical arm being in the sterile barrier installation pose, all joint links of the mechanical arm are in a following state:
all joint links of the mechanical arm that are protected by a sterile barrier are within a target height range from ground; and/or
an angle between every two adjacent joint links of the joint links of the mechanical arm is larger than a right angle.

14. The method for adjusting the mechanical arm configuration based on the single-cart single-arm surgical robot system according to any one of the preceding claims, wherein the target pose comprises an inverted placement pose; and
in response to the mechanical arm being in the inverted placement pose, an included angle between a parallelogram joint axis and ground is negative, and a pointing direction of a surgical instrument is opposite to a pointing direction from a cart base to a patient.

15. The method for adjusting the mechanical arm configuration based on the single-cart single-arm surgical robot system according to claim 14, wherein the included angle between the parallelogram joint axis and the ground ranges from -5° to -30°.

16. The method for adjusting the mechanical arm configuration based on the single-cart single-arm surgical robot system according to any one of the preceding claims, wherein the target pose comprises a forward placement pose; and
in response to the mechanical arm being in the forward placement pose, an included angle between a parallelogram joint axis and ground is positive, and a pointing direction of a surgical instrument is identical to a pointing direction from a cart base to a patient.

17. The method for adjusting the mechanical arm configuration based on the single-cart single-arm surgical robot system according to claim 16, wherein the included angle between the parallelogram joint axis and the ground ranges from 0° to 30°.

18. The method for adjusting the mechanical arm configuration based on the single-cart single-arm surgical robot system according to any one of the preceding claims, wherein in response to the movement of the mechanical arm in step S3, prompting an operating state in a manner of light or sound.

19. A single-cart single-arm surgical robot system, employing the method for adjusting the mechanical arm configuration based on the single-cart single-arm surgical robot system according to any one of claims 1 to 18, and comprising at least a mechanical arm, a control device, and a triggering device, wherein
the triggering device is configured to trigger the movement of the mechanical arm by employing the button-combination triggering mode;
the control device is configured to read the target pose corresponding to the button combination and calculate the planned movement trajectory of the mechanical arm; and
the mechanical arm is configured to be moved along the planned movement trajectory to reach the target pose.

20. The single-cart single-arm surgical robot system according to claim 19, wherein the target pose comprises a storage pose, a sterile barrier installation pose, and a specific placement pose; in a storage and transport stage, the triggering device is configured to employ the button-combination triggering mode to enable the control device to control the mechanical arm to reach the storage pose;
in a surgical preparation stage, the triggering device is configured to employ the button-combination triggering mode to enable the control device to control the mechanical arm to reach the sterile barrier installation pose for installing a sterile barrier;
in a beginning stage of a surgery, based on a type and manner of the surgery, the triggering device is configured to employ the button-combination triggering mode to enable the control device to control the mechanical arm to reach the specific placement pose, and the specific placement pose comprises an inverted placement pose and a forward placement pose; and
in an end stage of the surgery, the triggering device is configured to employ the button-combination triggering mode to enable the control device to control the mechanical arm to return to the storage pose.
